# EUROPEAN PATENT APPLICATION

(11) **EP 3 872 780 A1**
(43) Date of publication of application: **01.09.2021**
(21) Application number: 19875981.3
(22) Date of filing: 10.10.2019
(51) Int. Cl.: G08B 25/04, A61B 5/00, A61B 5/01, A61B 5/0245, A61B 5/113, A61B 5/1455, G08B 21/02, H04M 1/00, H04M 11/00

(54) **MONITORING DEVICE, MONITORING METHOD, AND MONITORING PROGRAM**

(30) Priority: 26.10.2018 JP 2018201796
(71) Applicant: Kyocera Corporation, Kyoto-shi, Kyoto 612-8501 (JP)
(72) Inventor: HIRANO Asao, Kyoto-shi, Kyoto 612-8501 (JP); HIGUCHI Takeshi, Kyoto-shi, Kyoto 612-8501 (JP)
(74) Representative: Viering, Jentschura & Partner mbB Patent- und Rechtsanwälte
(86) International application number: PCT/JP2019/040094
(87) International publication number: WO 2020/085108

(57) **Abstract**

A monitoring apparatus comprises a first acquisition unit, a second acquisition unit, a notification interface, and a controller. The first acquisition unit is configured to acquire biological information of a subject. The second acquisition unit is configured to acquire information about at least one of a position and an altitude of the subject. The notification interface is configured to notify the subject of predetermined information. The controller is configured to control the notification interface to notify the subject of breathing-related information, in the case of determining that the subject has risk related to breathing based on the biological information of the subject and the information about at least one of the position and the altitude of the subject.

## Description

### CROSS REFERENCE TO RELATED APPLICATION

This application claims priority to and the benefit of Japanese Patent Application No. 2018-201796 filed on October 26, 2018, the entire disclosure of which is incorporated herein by reference.

### TECHNICAL FIELD

The present disclosure relates to a monitoring apparatus, a monitoring method, and a monitoring program.

### BACKGROUND

Information provision systems that advise a user to stop movement based on biological information of the user and environmental information of the surroundings of the user are known (for example, see PTL 1). Moreover, exercise support apparatuses that indicate a predetermined breathing technique based on biological information of a user are known (for example, see PTL 2).

### CITATION LIST

### Patent Literature

PTL 1: JP 2013-220182 A
PTL 2: JP 2017-35327 A

### SUMMARY

A monitoring apparatus according to an embodiment of the present disclosure comprises a first acquisition unit, a second acquisition unit, a notification interface, and a controller. The first acquisition unit is configured to acquire biological information of a subject. The second acquisition unit is configured to acquire information about at least one of a position and an altitude of the subject. The notification interface is configured to notify the subject of predetermined information. The controller is configured to control the notification interface to notify the subject of breathing-related information, in the case of determining that the subject has risk related to breathing based on the biological information of the subject and the information about at least one of the position and the altitude of the subject.

A monitoring method according to an embodiment of the present disclosure comprises the following (1) to (4): (1) acquiring biological information of a subject; (2) acquiring information about at least one of a position and an altitude of the subject; (3) notifying the subject of predetermined information; and (4) controlling to notify the subject of breathing-related information, in the case of determining that the subject has risk related to breathing based on the biological information of the subject and the information about at least one of the position and the altitude of the subj ect.

A monitoring program according to an embodiment of the present disclosure causes a computer to execute the foregoing (1) to (4).

### BRIEF DESCRIPTION OF THE DRAWINGS

In the accompanying drawings:
FIG. 1 is a block diagram illustrating an example of the schematic structure of a monitoring apparatus according to an embodiment;
FIG. 2 is a diagram illustrating an example of the connection between the monitoring apparatus and a sensor;
FIG. 3 is a flowchart illustrating an example of a procedure for a monitoring method;
FIG. 4 is a conceptual diagram illustrating an example of a data structure stored in a memory;
FIG. 5 is a conceptual diagram illustrating an example of the data structure stored in the memory;
FIG. 6 is a flowchart illustrating an example of a procedure for determining biological information in stages;
FIG. 7 is a flowchart illustrating an example of a procedure for determining development of mountain sickness;
FIG. 8 is a flowchart illustrating an example of a mountain sickness determination procedure according to the present disclosure;
FIG. 9 is a block diagram illustrating an example of a structure in which the monitoring apparatus and a server are connected;
FIG. 10 is a block diagram illustrating an example of the internal structure of the server illustrated in FIG. 9;
FIG. 11 is a schematic diagram of a sensor of a first example used in the present disclosure;
FIG. 12 is a schematic diagram of a sensor of a second example used in the present disclosure;
FIG. 13 is a block diagram illustrating an example of the schematic structure of a monitoring apparatus according to another embodiment;
FIG. 14 is a flowchart illustrating an example of a procedure for a monitoring method;
FIG. 15 is a flowchart illustrating another example of the procedure for the monitoring method;
FIG. 16 is a diagram illustrating an example of a reference breathing rate corresponding to each altitude of a subject; and
FIG. 17 is a diagram illustrating an example of determination of risk related to the breathing of the subject.

### DETAILED DESCRIPTION

It is desirable to reduce risk related to the physical condition of a user in various situations to which the user is subjected. The present disclosure relates to provision of a monitoring apparatus, a monitoring method, and a monitoring program that can reduce risk related to the physical condition of a user. A monitoring apparatus, a monitoring method, and a monitoring program according to the present disclosure can reduce risk related to the physical condition of a user. An embodiment will be described in detail below, with reference to the drawings.

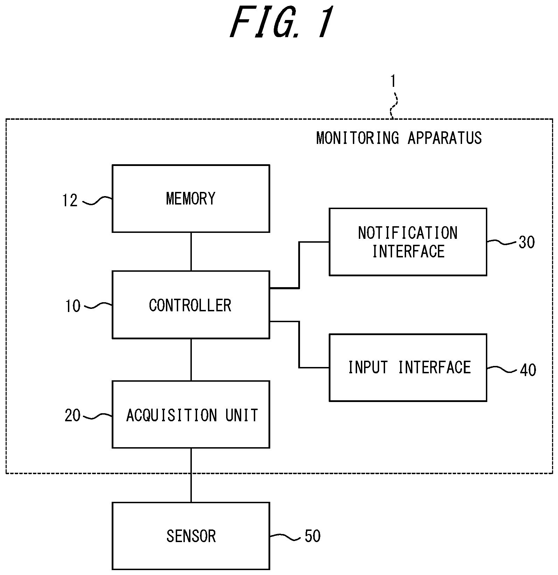

As illustrated in FIG. 1, a monitoring apparatus 1 according to an embodiment includes a controller 10, a memory 12, an acquisition unit 20, and a notification interface 30. The monitoring apparatus 1 may further include an input interface 40 that receives input from a user. The monitoring apparatus 1 is connected to an external sensor 50 via the acquisition unit 20. The monitoring apparatus 1 may include the sensor 50. The sensor 50 is worn by the user, and detects biological information of the user. The monitoring apparatus 1 can monitor changes in the body condition of the user, based on the biological information of the user detected by the sensor 50. A user whose biological information is detected by the sensor 50 is also referred to as a subject.

The controller 10 can control or manage each component of the monitoring apparatus 1. The controller 10 can transmit control information to each component of the monitoring apparatus 1, and acquire control information from each component. The controller 10 may include at least one processor such as a central processing unit (CPU) for executing a program defining a control procedure. The controller 10 may store the program and the like in the memory 12. The controller 10 may include the memory 12.

The at least one processor may include a single integrated circuit (IC) or a plurality of communicably-connected ICs or discrete circuits. The at least one processor may be implemented based on various known technologies. For example, the processor may include one or more circuits or units configured to perform one or more processes based on instructions stored in the memory 12 or a storage medium. The processor may be implemented as firmware for performing one or more processes. The firmware may be, for example, a discreet logic component.

The processor may include one or more processors, controllers, microprocessors, microcontrollers, application specific ICs, digital signal processors, programmable logic devices, or field programmable gate arrays. The processor may include any combination of these devices or configurations or combinations of other known devices and configurations.

The memory 12 may be semiconductor memory, magnetic memory, or the like. The memory 12 may store various information used in the controller 10, programs for operating the components in the monitoring apparatus 1, and the like. The memory 12 may function as working memory of the controller 10.

The acquisition unit 20 acquires the biological information of the subject from the sensor 50, and outputs the biological information to the controller 10. The controller 10 may store the biological information of the subject in the memory 12. The controller 10 may store the biological information of the subject in the memory 12, together with information about the time of detection by the sensor 50. The acquisition unit 20 may include a communication device. The communication device may be, for example, a communication interface of a local area network (LAN) or the like. The communication device may be communicably connected to an external apparatus, by wire or wirelessly. The acquisition unit 20 may be communicably connected to the sensor 50 by the communication device. The acquisition unit 20 may be included in the controller 10.

The sensor 50 may include a device that detects the breathing rate of the subject in a predetermined time period. The sensor 50 may include a device that detects the body temperature of the subject. The sensor 50 may include a device that detects the percutaneous oxygen saturation of the subject. The percutaneous oxygen saturation is also simply referred to as oxygen saturation. The percutaneous oxygen saturation is also referred to as SpO₂. S represents saturation, p represents pulse oximeter (pulse oximetry) or percutaneous, and O₂ represents oxygen. The sensor 50 may include a device that detects the pulse rate of the subject in a predetermined time period. The sensor 50 may include a device that detects the blood pressure of the subject. The sensor 50 may include a device that detects the blood flow amount of the subject. The sensor 50 may include a device that detects the electrical resistance of the body surface. The sensor 50 may include a device that detects brain waves of the subject.

The device that detects the blood flow amount will be described below. In the tissues of the living body, scattered light scattered from moving blood cells undergoes a frequency shift by a Doppler effect proportional to the moving speed of the blood cells in the blood. The frequency shift by the Doppler effect is also referred to as a Doppler shift. The controller 10 detects a beat signal generated as a result of light interference between scattered light from static tissues and scattered light from moving blood cells.

The beat signal represents intensity as a function of time. The controller 10 converts the beat signal into a power spectrum which represents power as a function of frequency. In the power spectrum of the beat signal, the Doppler shift frequency is proportional to the speed of blood cells. In the power spectrum of the beat signal, the power corresponds to the amount of blood cells. The controller 10 obtains the blood flow amount by multiplying the power spectrum of the beat signal by the frequency and integrating the multiplication result.

The notification interface 30 notifies the subject of information based on control information acquired from the controller 10. The notification interface 30 may include a display device. The display device may be, for example, a liquid crystal display, an organic electroluminescent (EL) display, or an inorganic EL display. The display device is not limited to such, and may be any other device. The notification interface 30 may display text, images, and the like on the display device, to notify the subject of the information based on the control information acquired from the controller 10. That is, the notification interface 30 notifies the subject of predetermined information.

The notification interface 30 may include a light source such as a light emitting diode (LED) or a halogen lamp. The notification interface 30 may notify the surroundings of the information based on the control information acquired from the controller 10, by illumination or blinking of the light source. The notification interface 30 may include a buzzer such as a piezoelectric buzzer or an electromagnetic buzzer, a speaker for generating predetermined sound, or the like. The notification interface 30 may notify to the surroundings of the information based on the control information acquired from the controller 10, by buzzer ringing, sound generation, or the like.

The input interface 40 may include physical keys such as a keyboard, or a touch panel. The input interface 40 is not limited to such, and may include any of various input devices.

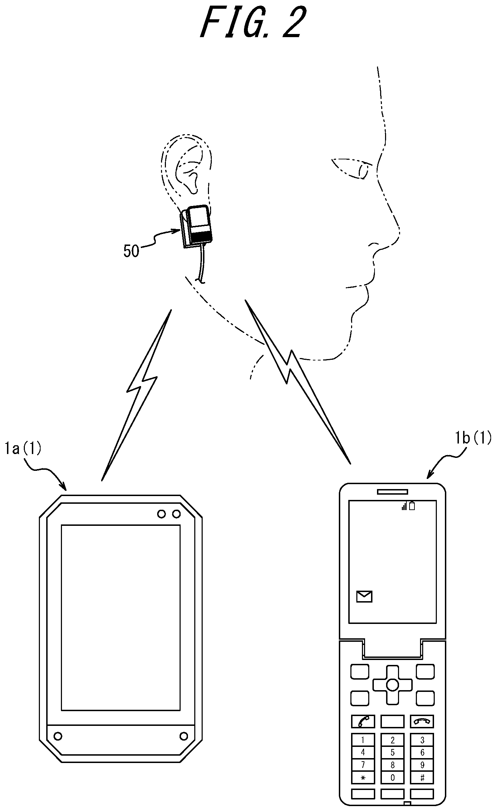

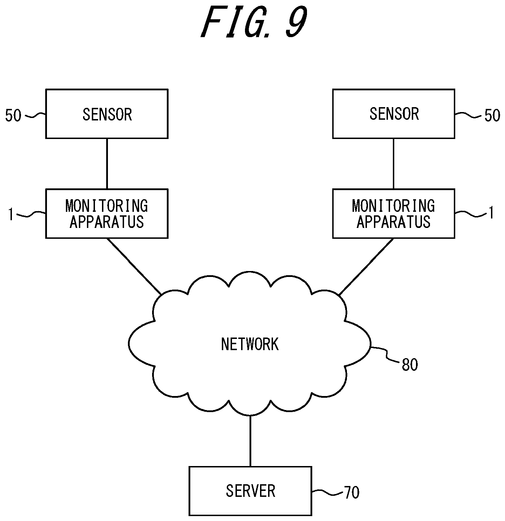

For example, the monitoring apparatus 1 may be a smartphone terminal 1a or a folding mobile phone terminal 1b, as illustrated in FIG. 2. The monitoring apparatus 1 is not limited to such, and may be any of various types of terminals or devices such as pendant type, wristband type, and eyeglass type. The monitoring apparatus 1 may be a general-purpose terminal or device capable of various functions, or a special-purpose terminal or device. The monitoring apparatus 1 and the monitoring method according to the embodiment may be implemented by executing, in a terminal or a device, a program including a procedure for monitoring the body condition of the subject. The program including the procedure for monitoring the body condition of the subject is also referred to as a monitoring program. The sensor 50 is connected to the monitoring apparatus 1 via a network 80 (see FIG. 9) that is wired, wireless, or a combination thereof, and information and the like are transmitted and received between the sensor 50 and the monitoring apparatus 1.

FIG. 2 illustrates an example of a pulse oximeter of a type worn on the subject's ear as the sensor 50. The pulse oximeter is not limited to a type worn on the ear, and may be of a type worn on other parts such as a finger. The sensor 50 is not limited to a pulse oximeter, and may be a device of any of other various types. The sensor 50 may be worn on such parts that do not interfere with the movement of the subject. The sensor 50 may be in a form that does not interfere with the movement of the subject. This improves user friendliness. In the present disclosure, the number of biological sensors provided as the sensor 50 is not limited to one, and a plurality of biological sensors may be provided. For example, the sensor 50 may include any combination of a breathing rate sensor, a SpO₂ sensor, a thermometer, a pulse sensor, a blood flow amount sensor, a blood pressure sensor, a heart rate sensor, and other appropriate biological sensors. These biological sensors may be separated between the sensor 50 and the monitoring apparatus 1 as appropriate. The monitoring apparatus 1 may include part of the biological sensors. For example, the breathing rate sensor may analyze vibrations in the pulse of the subject and detect the breathing rate. The breathing rate sensor may detect the movement of the human body, such as the abdomen, in contact with the human body or without contact, and detect the breathing rate. An example of the breathing sensor which does not contact the human body is an infrared sensor.

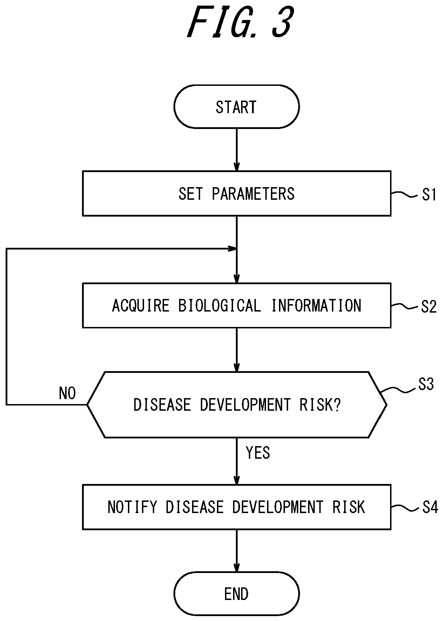

The monitoring apparatus 1 can monitor the condition of the subject according to the procedure of the flowchart illustrated in FIG. 3. As an example, suppose the monitoring apparatus 1 monitors the risk of the subject developing acute mountain sickness during climbing. Acute mountain sickness is also referred to as AMS. Acute mountain sickness is hereafter simply referred to as mountain sickness. Herein, the risk of developing each type of sickness such as mountain sickness or headache, ill health, injury, pain, malfunctioning of any part of the body, mental disturbance, or mental disorder is also referred to as disease development risk. What subsequently develops is referred to as the sickness, the disease, or the like. A person who has developed mountain sickness is unlikely to notice that he or she has developed mountain sickness. A person who has developed severe mountain sickness has a possibility of also developing brain edema, pulmonary edema, or the like concurrently, and slipping into critical condition. As a result of the subject being notified of the disease development risk before he or she develops mountain sickness or before the mountain sickness becomes severe, the subject can implement a coping measure to prevent development of severe mountain sickness. Thus, the health management of the subject can be supported, and the safety of the subject can be ensured easily.

The monitoring apparatus 1 is not limited to monitoring the risk of developing mountain sickness, and may monitor the risk of developing various diseases such as venous thrombosis. Venous thrombosis is also referred to as economy class syndrome. The monitoring apparatus 1 can monitor the risk of the subject developing a disease in the absence of subjective symptoms. The monitoring apparatus 1 can monitor the risk of the subject developing a disease that is difficult to recognize. By monitoring the disease development risk, the monitoring apparatus 1 can notify the subject of the risk before the subject develops the disease or before the disease becomes severe, and urge the subject to implement a coping measure. Consequently, the subject can prevent the development of the disease or prevent the disease from becoming severe.

The controller 10 sets parameters relating to control of each component in the monitoring apparatus 1 (step S1). The controller 10 may store the set parameters in the memory 12. The parameters may include a cycle for acquiring the biological information of the subject from the sensor 50. For example, the cycle for acquiring the biological information may be set in seconds or in minutes, or set to 1 hour or more. The parameters may include information specifying one or more types of biological information to be acquired from the sensor 50. The parameters may include one or more thresholds used for determination based on the biological information. The parameters are not limited to such, and may include various items. The controller 10 may set the parameters based on the defaults of the monitoring apparatus 1. The controller 10 may set the parameters based on input from the subject. The controller 10 may set the parameters based on the height, weight, age, sex, etc. of the subject. The controller 10 may set the parameters based on past biological information of the subject stored in the memory 12.

The controller 10 acquires the biological information of the subject from the sensor 50 (step S2). The type(s) of the biological information acquired may be set by the parameters. The biological information may include, for example, the breathing rate, SpO₂, body temperature, pulse rate, blood pressure, and/or blood flow amount of the subject.

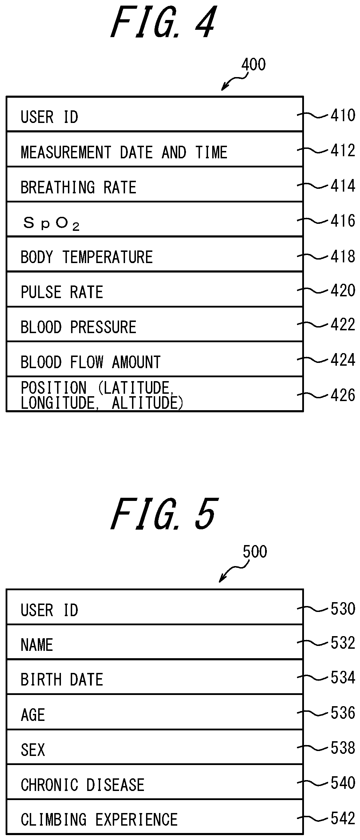

Data stored in the memory 12 will be described below, with reference to FIGS. 4 and 5. FIGS. 4 and 5 are conceptual diagrams illustrating an example of data structures 400 and 500 stored in the memory 12. In the present disclosure, the data structures stored in the memory 12 are not limited to the data structures 400 and 500 illustrated in FIGS. 4 and 5. One or more other data elements may be added as appropriate to the data structures 400 and 500 illustrated in FIGS. 4 and 5. One or more data elements may be omitted as appropriate from the data structures 400 and 500 illustrated in FIGS. 4 and 5.

As illustrated in FIG. 4, the data structure 400 stored in the memory 12 may include user ID 410, measurement date and time 412, breathing rate 414, SpO₂ 416, body temperature 418, pulse rate 420, blood pressure 422, blood flow amount 424, position 426, etc., as data elements. In the data structure 400, the user ID 410 and the measurement date and time 412 may be main keys. The user ID 410 may be data identifying the subject. The measurement date and time 412 may be data about the date and time of detection of the biological information of the subject. The breathing rate 414, the SpO₂ 416, the body temperature 418, the pulse rate 420, the blood pressure 422, and the blood flow amount 424 may be data about the breathing rate, SpO₂, body temperature, pulse rate, blood pressure, and blood flow amount detected from the subject, respectively. The position 426 may be data about the current position of the subject. The current position of the subject may be expressed by latitude, altitude, longitude, or any combination thereof. The data elements included in the data structure 400 may be data about biological information of the subject.

As illustrated in FIG. 5, the data structure 500 stored in the memory 12 may include user ID 530, name 532, birth date 534, age 536, sex 538, chronic disease 540, climbing experience 542, etc., as data elements. In the data structure 500, the user ID 530 may be a main key. The data elements included in the data structure 500 may be items about personal information of the subject. The name 532, the birth date 534, the age 536, and the sex 538 may be data about the name, birth date, age, and sex of the subject, respectively. The chronic disease 540 may be data about the name of any disease with which the subject was diagnosed, symptoms which the subject recognized, and the like. The climbing experience 542 may be data about the number of years of climbing experience of the subject, the names and/or number of mountains climbed, and the like.

The data elements included in the data structures 400 and 500 may be replaced with each other, or combined into one data structure 400 or 500.

The controller 10 determines whether the subject has disease development risk, based on at least one piece of biological information (step S3). The controller 10 may determine that the subject has disease development risk, in the case where the SpO₂ of the subject is less than a predetermined value. The predetermined value concerning SpO₂ may be, for example, 85 %. The predetermined value is, however, not limited to such, and may be determined as appropriate. The controller 10 may determine that the subject has disease development risk, in the case where the body temperature of the subject is greater than or equal to a predetermined value. The predetermined value concerning body temperature may be, for example, 37.0 °C. The predetermined value is, however, not limited to such, and may be determined as appropriate. The controller 10 may determine that the subject has disease development risk, in the case where the breathing rate of the subject is outside a predetermined range. That is, the controller 10 may determine that the subject has disease development risk, in the case where the breathing rate of the subject is excessively low or excessively high. The predetermined range concerning breathing rate may be, for example, a range of 10 to 25 per minute. The predetermined range is, however, not limited to such, and may be determined as appropriate.

The controller 10 may determine whether the subject has disease development risk, based on results of comparing a plurality of pieces of biological information with respective thresholds. The controller 10 may calculate a score quantifying the disease development risk, based on at least one piece of biological information. The controller 10 may determine that the subject has disease development risk, in the case where the score is greater than or equal to a predetermined value or in the case where the score is less than or equal to a predetermined value. The predetermined value concerning score may be determined as appropriate.

The controller 10 may determine whether the subject has disease development risk, based on a result of comparing biological information acquired a predetermined time or more ago and biological information acquired less than the predetermined time ago, which are stored in the memory 12. The controller 10 may determine that the subject has disease development risk, in the case where the difference between the biological information acquired the predetermined time or more ago and the biological information acquired less than the predetermined time ago is greater than or equal to a predetermined value. That is, the controller 10 may determine that the subject has disease development risk, in the case where the amount of change of biological information is greater than or equal to the predetermined value. The predetermined value concerning the amount of change of biological information may be determined as appropriate.

In the case where the subject does not have disease development risk (step S3: NO), the controller 10 returns to step S2. In the case where the subject has disease development risk (step S3: YES), the controller 10 causes the notification interface 30 to notify the subject of information about the disease development risk (step S4). The information about the disease development risk may include the determination result that the subject has disease development risk. The information about the disease development risk may include information about a coping measure for the disease development risk of mountain sickness. For example, the coping measure may be to urge the subject to use a breathing technique that improves the symptoms of mountain sickness, or to urge the subject to descend the mountain. The coping measure for mountain sickness is not limited to such, and may be any of various measures. The controller 10 may output control information including the contents of the notification to the notification interface 30. The notification interface 30 may notify the subject of the contents based on the control information, or notify a person in the vicinity of the subject of the contents. After step S4, the controller 10 ends the procedure of the flowchart in FIG. 3. After step S4, the controller 10 may return to step S1 or S2.

Examples of the coping measure for the disease development risk of mountain sickness include information regarding a massage technique, information regarding fluid intake, information regarding resting time, and information regarding resting attitude.

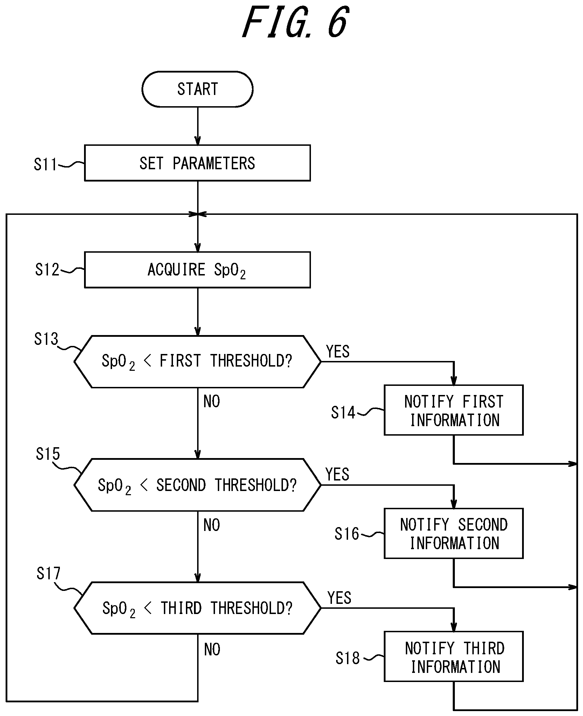

The monitoring apparatus 1 can notify the subject of information in stages based on the biological information of the subject, according to the procedure of the flowchart illustrated in FIG. 6. The monitoring apparatus 1 may notify the subject of information in stages based on comparison results of the SpO₂ of the subject and a plurality of thresholds. The monitoring apparatus 1 may notify the subject of information in stages based on comparison between various biological information other than SpO₂ and a plurality of thresholds. The order of the steps in the flowchart illustrated in FIG. 6 may be changed as appropriate.

The controller 10 sets parameters relating to control of each component in the monitoring apparatus 1 (step S11). The controller 10 may set a threshold to be compared with the SpO₂ of the subject, as a parameter. The controller 10 may set a first threshold, a second threshold, and a third threshold. The number of thresholds is not limited to three, and may be two or less, or four or more. The first threshold is less than the second threshold. The second threshold is less than the third threshold. For example, the first threshold, the second threshold, and the third threshold may be respectively set to 70 %, 80 %, and 90 %. These threshold values are based on the result of demonstration experiments conducted by the inventors at high altitude. The thresholds are not limited to these values, and may be set to other values.

The controller 10 acquires the SpO₂ of the subject from the sensor 50 (step S12).

The controller 10 determines whether the SpO₂ is less than the first threshold (step S13). In the case where the SpO₂ is less than the first threshold (step S13: YES), the controller 10 causes the notification interface 30 to notify first information (step S14). The first information may include information urging the subject to stop climbing and descend the mountain. The first information may include other information. After step S14, the controller 10 returns to step S12.

In the case where the SpO₂ is not less than the first threshold (step S13: NO), the controller 10 determines whether the SpO₂ is less than the second threshold (step S15). In the case where the SpO₂ is less than the second threshold (step S15: YES), the controller 10 causes the notification interface 30 to notify second information (step S16). The second information may include information urging the subject to use a breathing technique that prevents development of mountain sickness. The breathing technique may be, for example, a pressure breathing technique or an abdominal breathing technique. The breathing technique is not limited to such, and may be any of other various techniques. The second information may include information indicating a specific procedure of the breathing technique. The second information is not limited to these information, and may include other information. After step S16, the controller 10 returns to step S12. The second information may include information regarding a massage technique, information regarding fluid intake, information regarding resting time, information regarding resting attitude, etc.

In the case where the SpO₂ is not less than the second threshold (step S15: NO), the controller 10 determines whether the SpO₂ is less than the third threshold (step S17). In the case where the SpO₂ is less than the third threshold (step S17: YES), the controller 10 causes the notification interface 30 to notify third information (step S18). The third information may include information urging the subject to rest in order to prevent development of mountain sickness. The third information may include information urging the subject to exercise. The third information may include information urging the subject to receive hydration. The third information is not limited to these information, and may include other information. After step S18, the controller 10 returns to step S12. In the case where the SpO₂ is not less than the third threshold (step S17: NO), the controller 10 returns to step S12. The third information may include information regarding a massage technique, information regarding fluid intake, information regarding resting time, information regarding resting attitude, etc.

The monitoring apparatus 1 can notify information suitable for the condition of the subject, by determining the biological information based on the thresholds in stages.

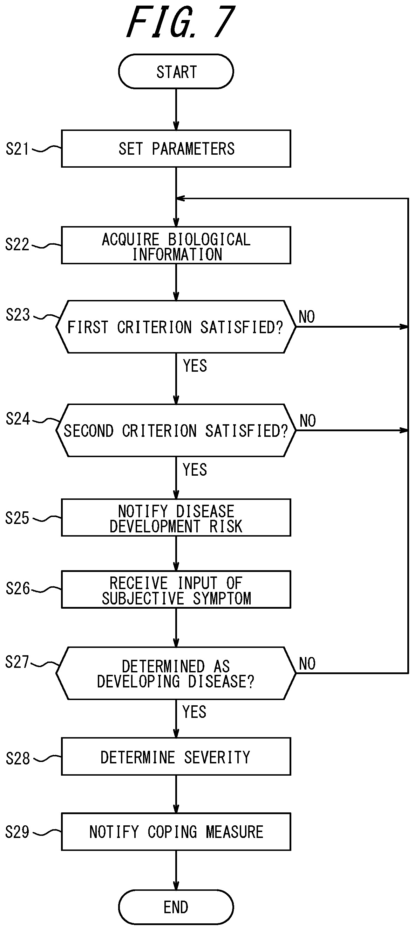

The monitoring apparatus 1 can perform a more detailed determination of whether the subject has disease development risk based on the biological information of the subject, according to the procedure of the flowchart illustrated in FIG. 7.

The controller 10 sets parameters relating to control of each component in the monitoring apparatus 1 (step S21). The controller 10 may perform a procedure that is the same as or similar to step S1 in FIG. 3.

The controller 10 acquires the biological information of the subject from the sensor 50 (step S22). The controller 10 may perform a procedure that is the same as or similar to step S2 in FIG. 3.

The controller 10 determines whether the biological information satisfies a first criterion (step S23). The first criterion may include the breathing rate of the subject being greater than or equal to a predetermined threshold. In the case where the breathing rate of the subject is greater than or equal to the predetermined threshold, there is a possibility that the subject is in a state of hyperpnea. The first criterion may include the breathing rate of the subject being less than a predetermined threshold. In the case where the breathing rate of the subject is less than the predetermined threshold, there is a possibility that the subject is in a state of apnea. The first criterion may include the SpO₂ of the subject being less than a predetermined threshold. In the case where the SpO₂ of the subject is less than the predetermined threshold, there is a possibility that the subject is in a state of hypoxia. The first criterion may include the body temperature of the subject being greater than or equal to a predetermined threshold. In the case where the body temperature of the subject is greater than or equal to the predetermined threshold, there is a possibility that the subject is in a state of feverishness. The first criterion may include the body temperature of the subject being less than a predetermined threshold. In the case where the body temperature of the subject is less than the predetermined threshold, there is a possibility that the subject is in a state of hypothermia. The first criterion is not limited to such, and may include various conditions, and may be a combination of a plurality of conditions.

In the case where the biological information does not satisfy the first criterion (step S23: NO), the controller 10 returns to step S22. In the case where the biological information satisfies the first criterion (step S23: YES), the controller 10 determines whether the biological information satisfies a second criterion (step S24). The second criterion may include the same condition as that included in the first criterion, or include a different condition from that included in the first criterion. The second criterion may include the pulse rate of the subject being greater than or equal to a predetermined threshold. In the case where the pulse rate of the subject is greater than or equal to the predetermined threshold, there is a possibility that the subject is in a state of hypoxia. The second criterion may include a condition relating to the autonomic nerve state. The autonomic nerve state can be represented by the balance between the strength of sympathetic nerve activity and the strength of parasympathetic nerve activity. The second criterion may include the difference between the strength of sympathetic nerve activity and the strength of parasympathetic nerve activity being greater than or equal to a predetermined value. The controller 10 may determine the autonomic nerve state based on the biological information of the subject. The controller 10 may determine the autonomic nerve state based on the variation in heartbeat. The controller 10 may determine the autonomic nerve state based on the variation in the electrical resistance of the body surface of the subject.

In the case where the biological information does not satisfy the second criterion (step S24: NO), the controller 10 returns to step S22. In the case where the biological information satisfies the second criterion (step S24: YES), the controller 10 causes the notification interface 30 to notify the subject of information about the disease development risk (step S25). The controller 10 may cause the notification interface 30 to notify information urging the subject to input subjective symptoms.

The controller 10 receives input of subjective symptoms from the subject by the input interface 40 (step S26). The controller 10 may cause the notification interface 30 to produce a display requesting the subject to answer questions based on the Lake Louise Score (LLS). The questions which the subject is requested to answer are not limited to the LLS-based questions, and may be other various questions. Examples of the subjective symptoms of the subject include physical pain such as headache, decreased appetite, fatigue or a feeling of weariness, dizziness or lightheadedness, and a sleeping state. In the present disclosure, the controller 10 may cause the notification interface 30 to produce a display requesting the subject to answer questions other than the LLS-based questions.

The controller 10 determines whether the subject is developing mountain sickness, based on the subjective symptoms of the subject (step S27). The controller 10 may calculate a score quantifying the subjective symptoms of the subject. The controller 10 may calculate a score quantifying the answers of the subject to the LLS-based questions. The controller 10 may determine whether the subject is developing mountain sickness, based on the calculated score. The controller 10 may determine the severity of the mountain sickness developed by the subject, based on the calculated score.

In the case where the controller 10 does not determine that the subject is developing mountain sickness (step S27: NO), the controller 10 returns to step S22. In this case, the controller 10 may cause the notification interface 30 to notify that the likelihood of the subject developing mountain sickness is low. Given that the subject satisfies the second criterion (step S24), for example, the controller 10 may determine that the subject is predicted to develop mountain sickness. Given that the subject satisfies the second criterion (step S24), for example, the controller 10 may cause the notification interface 30 to notify a coping measure for the disease development risk.

In the case where the controller 10 determines that the subject is developing mountain sickness (step S27: YES), the controller 10 determines the severity of the mountain sickness based on the biological information (step S28). The controller 10 may determine the severity of the mountain sickness based on the blood pressure or blood flow amount of the subject. For example, the controller 10 may determine the severity of the mountain sickness to be higher when the blood pressure of the subject is lower. For example, the controller 10 may determine the severity of the mountain sickness to be higher when the blood flow amount to the brain of the subject is larger. The controller 10 may determine the severity of the mountain sickness based on other various biological information, without being limited to these biological information. The controller 10 may determine the severity of the mountain sickness in stages, by comparing the biological information with a plurality of thresholds as in the procedure of the flowchart in FIG. 6.

The controller 10 causes the notification interface 30 to notify information about the coping measure for the subject based on the determination result on the severity of the mountain sickness (step S29). The coping measure may be based on the severity of the mountain sickness. For example, the coping measure may be to urge the subject to descend the mountain, to urge the subject or another person to report, or to urge the subject to use a breathing technique that improves the symptoms of mountain sickness. The coping measure for mountain sickness is not limited to such, and may be any of other various measures. After step S29, the controller 10 ends the procedure of the flowchart in FIG. 7.

In the flowchart in FIG. 7, any one of steps S23 and S24 may be omitted. That is, the controller 10 may advance to step S25 to notify the disease development risk, in the case where one of the first criterion and the second criterion is satisfied.

The monitoring apparatus 1 may determine whether the subject has risk of developing economy class syndrome. The monitoring apparatus 1 may determine whether the subject has risk of developing economy class syndrome, based on the blood flow amount of the subject. The monitoring apparatus 1 may determine whether the subject has risk of developing economy class syndrome based on other various biological information, without being limited to the blood flow amount of the subject. In the case where the subject has risk of developing economy class syndrome, the monitoring apparatus 1 may notify a coping measure to urge the subject to exercise or to urge the subject to receive hydration. The coping measure for economy class syndrome is not limited to such, and may be any of other various measures.

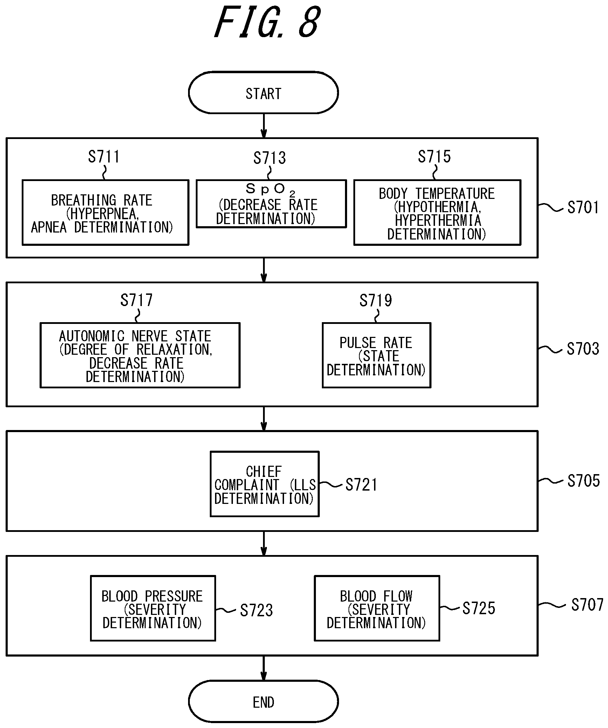

A specific mountain sickness determination process will be further described below, with reference to FIG. 8. FIG. 8 is a flowchart of mountain sickness determination according to the present disclosure. The measurement order in the flowchart in FIG. 8 is an example, and may be changed as appropriate.

The controller 10 performs a determination based on the breathing rate, SpO₂, or body temperature of the subject (step S701). The controller 10 may determine whether the subject is in a state of hyperpnea or apnea, based on the breathing rate of the subject (step S711). The controller 10 may determine the SpO₂ decrease rate, based on the SpO₂ of the subject (step S713). The controller 10 may determine whether the subject is in a state of hypothermia or hyperthermia, based on the body temperature of the subject (step S715). The steps included in step S701 may be performed in a different order, and any of the steps may be skipped as appropriate. In the case where at least one determination of the determinations in the steps included in step S701 satisfies the condition, the controller 10 may advance to step S703. The controller 10 may store the determination result in each step included in step S701 or a value obtained by converting the determination result into a score, in the memory 12. After storing the determination result in the memory 12, the controller 10 may advance to step S703 regardless of whether the determination in each step included in step S701 satisfies the condition.

The controller 10 performs the determination based on the autonomic nerve state or pulse rate of the subject (step S703). The controller 10 may determine the degree of relaxation or the deterioration rate of the autonomic nerve state, based on the autonomic nerve state of the subject (step S717). The controller 10 may determine the condition of the subject, based on the pulse rate of the subject (step S719). The steps included in step S703 may be performed in a different order, and any of the steps may be skipped as appropriate. In the case where at least one determination of the determinations in the steps included in step S703 satisfies the condition, the controller 10 may advance to step S705. The controller 10 may store the determination result in each step included in step S703 or a value obtained by converting the determination result into a score, in the memory 12. After storing the determination result in the memory 12, the controller 10 may advance to step S705 regardless of whether the determination in each step included in step S703 satisfies the condition.

The controller 10 performs the determination based on the chief complaint of the subject (step S705). The controller 10 may perform LLS determination based on the chief complaint of the subject (step S721). The controller 10 may advance to step S707 in the case where the determination in step S721 satisfies the condition. The controller 10 may store the determination result in step S721 in the memory 12, or convert the determination result into a score and store the score in the memory 12. After storing the determination result in the memory 12, the controller 10 may advance to step S707 regardless of whether the determination in step S721 satisfies the condition.

The controller 10 performs the determination based on the blood pressure or blood flow of the subject (step S707). The controller 10 may determine the severity of the subject based on the blood pressure of the subject (step S723). The controller 10 may determine the severity of the subject based on the blood flow of the subject (step S725). The steps included in step S707 may be performed in a different order, and any of the steps may be skipped as appropriate. In the case where at least one determination of the determinations in the steps included in step S707 satisfies the condition, the controller 10 may determine that the subject has severe mountain sickness, and end the procedure of the flowchart in FIG. 8. The controller 10 may store the determination result in each step included in step S707 or a value obtained by converting the determination result into a score, in the memory 12. After storing the determination result in the memory 12, the controller 10 may end the procedure of the flowchart in FIG. 8 regardless of whether the determination in each step included in step S707 satisfies the condition. The controller 10 may advance to the next step after performing the steps included in steps S701, S703, S705, and S707.

As illustrated in FIG. 9, each monitoring apparatus 1 connected to a sensor 50 may be communicably connected to a server 70 via the network 80. The number of servers 70 is not limited to one, and may be two or more. The number of monitoring apparatuses 1 and the number of sensors 50 are each not limited to two, and may be one, or three or more. The number of sensors 50 connected to one monitoring apparatus 1 is not limited to one, and may be two or more. In the present disclosure, the sensor 50 may be connected to the server 70 without the monitoring apparatus 1 therebetween. The network 80 may be a wired network, a wireless network, or a combination thereof.

The server 70 can acquire biological information of a subject detected by a sensor 50, from each monitoring apparatus 1. The server 70 may acquire biological information of a plurality of subjects. The server 70 may analyze the acquired biological information using various calculations such as averaging. The server 70 may analyze the acquired biological information by a statistical technique. The server 70 may transmit the analysis result of the biological information to each monitoring apparatus 1. The monitoring apparatus 1 may set parameters relating to control of each component in the monitoring apparatus 1, based on the analysis result of the biological information. The server 70 may determine the disease development risk of the subject based on the biological information of the subject, and transmit the determination result to the monitoring apparatus 1. The monitoring apparatus 1 may notify information based on the determination result of the disease development risk by the server 70. The server 70 may generate information about a coping measure for the subject based on the biological information of the subject, and transmit the generated information to the monitoring apparatus 1. The monitoring apparatus 1 may notify the subject of the information about the coping measure generated by the server 70.

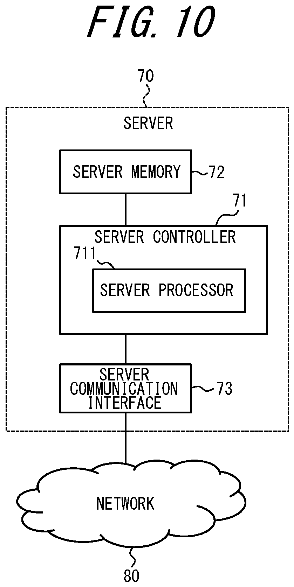

The internal structure of the server 70 will be described below, with reference to FIG. 10. FIG. 10 is a block diagram illustrating an example of the internal structure of the server 70 illustrated in FIG. 9.

The server 70 includes a server controller 71, a server communication interface 73, and a server memory 72.

The server controller 71 is a processor that controls and manages the whole server 70, e.g. each functional block in the server 70. The server controller 71 includes a processor such as a CPU that executes a program defining a control procedure. Such a program is, for example, stored in the server memory 72 or an external storage medium connected to the server 70.

The server controller 71 includes at least one server processor 711 to provide control and throughput for executing various functions, as described in more detail below.

In various embodiments, at least one server processor 711 may be implemented as a single integrated circuit (IC), or as a plurality of ICs and/or discrete circuits communicably connected to one another. At least one server processor 711 can be implemented according to various known technologies.

In one embodiment, the server processor 711 includes, for example, one or more circuits or units configured to perform one or more data calculation procedures or processes by executing instructions stored in related memory. In another embodiment, the server processor 711 may be firmware configured to perform one or more data calculation procedures or processes. The firmware may be, for example, a discrete logic component.

In various embodiments, the server processor 711 may include one or more processors, controllers, microprocessors, microcontrollers, application specific integrated circuits (ASICs), digital signal processors, programmable logic devices, field programmable gate arrays, or any combination of these devices or structures, or any combination of other known devices or structures, to perform the functions of the server controller 71 described below.

The server memory 72 may be semiconductor memory, magnetic memory, or the like. The server memory 72 stores various information, programs for operating the server 70, and the like. The server memory 72 may function as working memory.

Data stored in the server memory 72 may be the same as the data elements in the data structures 400 and 500 illustrated in FIGS. 4 and 5. Besides the data illustrated in FIGS. 4 and 5, the server memory 72 may store various control programs, application programs, and the like. The server 70 may acquire data from separate subjects each using a sensor 50. That is, the server memory 72 in the server 70 may store the data illustrated in FIGS. 4 and 5 for each of a plurality of subjects.

Some of the information stored in the server memory 72 may be acquired from a monitoring apparatus 1 or a sensor 50. Some of the information stored in the server memory 72 may be acquired from another server 70 connected to the server 70 via the network 80.

The server 70 may acquire a result of determining whether a subject has disease development risk, from each monitoring apparatus 1. The server 70 may acquire disease development risk determination results for a plurality of subjects. The server 70 may analyze each determination result by a statistical method. The server 70 may transmit a result of analyzing the determination result to the monitoring apparatus 1. The server 70 may generate information to be notified to the subject based on the result of analyzing the determination result, and transmit the generated information to the monitoring apparatus 1.

As a result of the server 70 analyzing biological information of a plurality of subjects, the disease development risk determination accuracy in the monitoring apparatus 1 can be enhanced.

The monitoring apparatus 1 may include a position sensor for detecting the position of the monitoring apparatus 1. The position sensor may acquire the position information of the monitoring apparatus 1 based on, for example, global navigation satellite system (GNSS) technologies including satellite navigation systems such as Global Positioning System (GPS), GLONASS, Galileo, and Quasi-Zenith Satellite System (QZSS). The monitoring apparatus 1 may transmit the position information of the monitoring apparatus 1 to the server 70. The position information includes latitude, longitude, and altitude information.

The server 70 may determine whether the monitoring apparatus 1 is located in a predetermined range, based on the position information of the monitoring apparatus 1. The server 70 may analyze biological information of a subject or a determination result based on the biological information acquired from each monitoring apparatus 1 located in the predetermined range, by a statistical method. By analyzing whether the determination results in the predetermined range exhibit bias, the possibility that an event which affects the determination results is occurring in the predetermined range can be detected. Consequently, the disease development risk of each subject present in the predetermined range can be determined with higher accuracy.

### (First example of sensor 50)

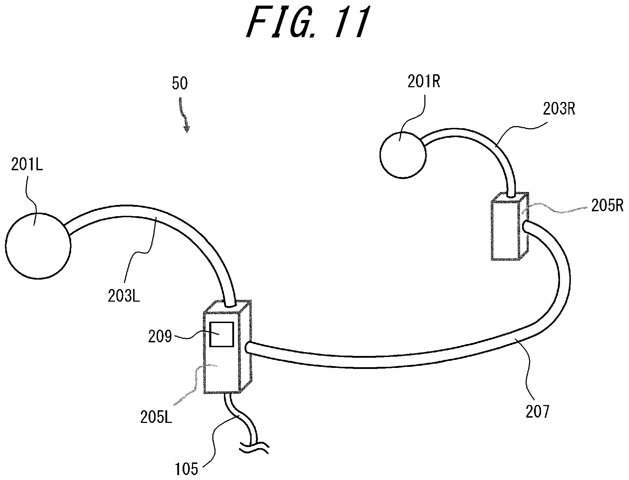

The sensor 50 used in the present disclosure is not limited to that illustrated in FIG. 2. A sensor 50 of a first example used in the present disclosure will be described below, with reference to FIG. 11.

As illustrated in FIG. 11, the sensor 50 includes a holder 203L held on the auricle of the left ear of the subject. The sensor 50 also includes a housing 205L provided at the holder 203L on the occipital region side of the left ear of the subject. The sensor 50 also includes a measurement unit 201L provided at the holder 203L on the face side of the left ear of the subject. The sensor 50 also includes a sensor communication interface 209 having a cable 105 to be connected to a smartphone. In the example in FIG. 11, the sensor communication interface 209 is covered by the housing 205L. Accordingly, the cable 105 extends from the housing 205L. The cable 105 may be provided at a part other than the housing 205L.

As illustrated in FIG. 11, the sensor 50 includes a holder 203R held on the auricle of the right ear of the subject. The sensor 50 also includes a housing 205R provided at the holder 203R on the occipital region side of the right ear of the subject. The sensor 50 also includes a measurement unit 201R provided at the holder 203R on the face side of the right ear of the subject. At least one of the measurement units 201L and 201R is located, for example, at the temporal region. At least one of the measurement units 201L and 201R may be located at a part other than the temporal region.

The sensor 50 illustrated in FIG. 11 also includes a connector 207 connecting the housings 205L and 205R. The holder 203L, the holder 203R, and the connector 207 may be made of plastic, rubber, cloth, paper, resin, iron, or other material, or any combination thereof. At least one of the housings 205L and 205R is located, for example, at the mastoid region. At least one of the housings 205L and 205R may be located at a part other than the mastoid region.

The sensor 50 in the present disclosure may have, for example, a structure in which at least one of the measurement unit 201R and the housing 205R is omitted from the structure illustrated in FIG. 11. The sensor 50 in the present disclosure may have, for example, a structure in which at least one of the measurement unit 201L and the housing 205L is omitted from the structure illustrated in FIG. 11. The sensor 50 in the present disclosure may have, for example, a structure in which the measurement unit 201R and the housing 205L are omitted from the structure illustrated in FIG. 11. The sensor 50 in the present disclosure may have, for example, a structure in which the measurement unit 201L and the housing 205R are omitted from the structure illustrated in FIG. 11.

At least one of the measurement units 201L and 201R includes a breathing rate sensor, a SpO₂ sensor, a body temperature sensor, a pulse rate sensor, a blood flow amount sensor, a pulse wave sensor, a heart rate sensor, and the like.

### (Second example of sensor 50)

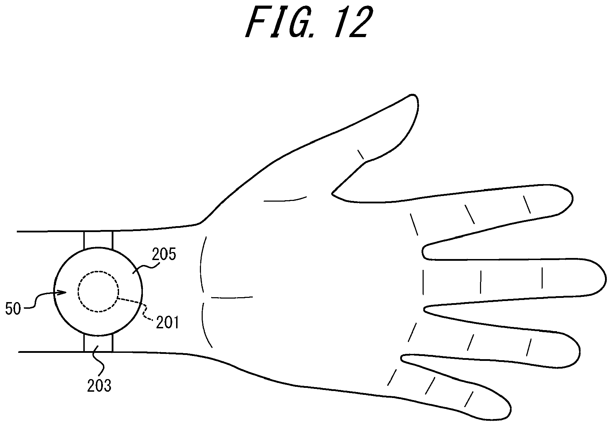

A sensor 50 of a second example used in the present disclosure will be described below, with reference to FIG. 12. As illustrated in FIG. 12, the sensor 50 of the second example is of wristwatch type. The sensor 50 includes a measurement unit 201 located on the side of the body surface of the subject, and a holder 203 worn on the arm of the subject. The measurement unit 201 may be in contact with the body surface of the subject. The measurement unit 201 may include a breathing rate sensor, a SpO₂ sensor, a body temperature sensor, a pulse rate sensor, a blood flow amount sensor, a pulse wave sensor, a heart rate sensor, and the like. The holder 203 may be a belt wrapped around the arm of the subject, or have any other form that can be worn by the subject. The holder 203 may be made of plastic, rubber, cloth, paper, resin, iron, or other material, or any combination thereof.

The sensor 50 used in the present disclosure is not limited to the foregoing forms. For example, the sensor 50 may be included in any appropriate object such as a wristwatch, a cane, a flashlight, a hat, clothes, pants, shoes, eyeglasses, a helmet, a rucksack, a bag, a water bottle, a compass, a bicycle, an automobile, or a motorcycle. The sensor 50 in the present disclosure may have any appropriate shape such as a clip shape or a band shape.

In the sensor 50 in the present disclosure, a breathing rate sensor, a SpO₂ sensor, a body temperature sensor, a pulse rate sensor, a blood flow amount sensor, a pulse wave sensor, a heart rate sensor, and the like may be included in one device, or separated between a plurality of devices.

The monitoring apparatus 1 in the present disclosure may monitor the physical condition of the user as the subject, by appropriately using information other than biological information, such as position information, atmospheric temperature information, humidity information, weather information, time information, headache frequency, moving distance, and fluid intake.

### (Notification destination of monitoring apparatus 1)

In the present disclosure, for example, in the case where the physical condition of the user changes and the determination in step S13, S15, or S17 in FIG. 6 results in YES to thus indicate that the subject is at risk of having symptoms of mountain sickness or the like, the monitoring apparatus 1 or the sensor 50 may notify not only the user but also a person other than the user, a device, a server, and the like. In the case where the determination in step S13, S15, or S17 in FIG. 6 results in YES to thus indicate that the subject is at risk of having symptoms of mountain sickness or the like, the monitoring apparatus 1 may notify, for example, the first information, the second information, or the third information in FIG. 6 to a person other than the user in a group to which the user using the sensor 50 belongs, a family or a friend of the user using the sensor 50, a medical institution, an administrative body, or the like. The person other than the user in the group to which the user using the sensor 50 belongs may be, for example, a leader of the group to which the user using the sensor 50 belongs. For example, in the case where the user using the sensor 50 is traveling, the leader may be a tour guide. In the case where the determination in step S13, S15, or S17 in FIG. 6 or step S27 results in YES to thus indicate that the subject is at some risk of having symptoms of mountain sickness or the like, the monitoring apparatus 1 may notify, for example, information about the risk to a server, a security alarm, a personal computer, a smartphone, a mobile phone, etc. of a person other than the user in a group to which the user using the sensor 50 belongs, a family or a friend of the user using the sensor 50, a medical institution, an administrative body, or the like.

Each sensor 50 described above may be connected to not only a smartphone but also a mobile phone, a music player, a game machine, a personal computer, a server, a tablet terminal, or the like.

For example, the presently disclosed technique may be used in situations in which the user is climbing, jogging, running, walking, driving an automobile, driving a motorcycle, driving a bicycle, flying an airplane, steering a ship, or sightseeing. The presently disclosed technique may be used in situations in which the user is driving or riding a train, a bus, an automobile, a motorcycle, a bicycle, an airplane, or a ship. Situations in which the presently disclosed technique is used are not limited to such.

### (Monitoring apparatus according to another embodiment)

A monitoring apparatus according to another embodiment will be described below.

The monitoring apparatus according to another embodiment monitors, for example, the breathing rate of a user during climbing or the like. In the case where the monitoring apparatus determines that the subject has risk related to breathing such as mountain sickness, the monitoring apparatus notifies the subject of breathing-related information such as urging the subject to breath. The monitoring apparatus according to this embodiment will be described in detail below.

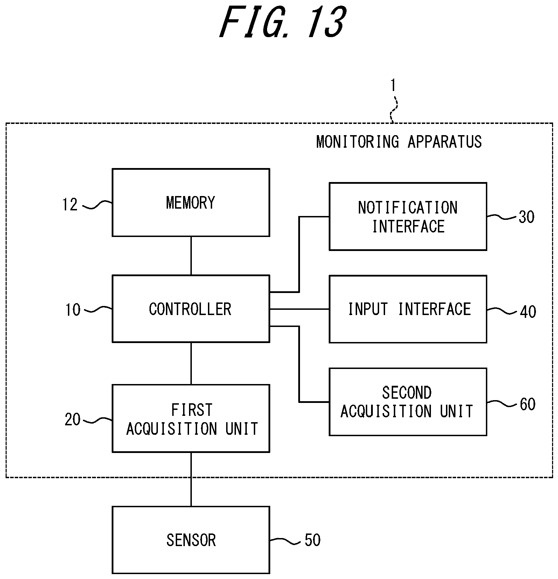

As illustrated in FIG. 13, a monitoring apparatus 1 according to an embodiment is the same as the monitoring apparatus 1 illustrated in FIG. 1 in that it includes a controller 10 and a notification interface 30. The monitoring apparatus 1 illustrated in FIG. 13 may further include a memory 12, a first acquisition unit 20, and an input interface 40, as in the monitoring apparatus 1 illustrated in FIG. 1. The first acquisition unit 20 in the monitoring apparatus 1 illustrated in FIG. 13 may be a functional unit same as or similar to the acquisition unit 20 in the monitoring apparatus 1 illustrated in FIG. 1. The acquisition unit 20 illustrated in FIG. 1 is referred to as "first acquisition unit 20" in the following description, for convenience's sake. That is, the first acquisition unit 20 acquires biological information of the subject. The biological information may include at least one of the breathing rate, oxygen saturation, pulse rate, and body temperature of the subject.

The monitoring apparatus 1 illustrated in FIG. 13 is connected to an external sensor 50 via the first acquisition unit 20, as in the monitoring apparatus 1 illustrated in FIG. 1. The monitoring apparatus 1 illustrated in FIG. 13 can monitor changes in the body condition of the user based on the biological information of the user detected by the sensor 50, as in the monitoring apparatus 1 illustrated in FIG. 1. These functional units may be the same as the functional units included in the monitoring apparatus 1 illustrated in FIG. 1. Description of parts same as or similar to those described above with regard to the monitoring apparatus 1 illustrated in FIG. 1 is simplified or omitted as appropriate.

The monitoring apparatus 1 illustrated in FIG. 13 further includes a second acquisition unit 60. In the monitoring apparatus 1 according to the embodiment, the second acquisition unit 60 acquires information about at least one of the position and altitude of the subject. The second acquisition unit 60 may be a position sensor for detecting the position of the monitoring apparatus 1, such as a GPS module. The position sensor may acquire the position information of the monitoring apparatus 1 based on, for example, GNSS technologies including satellite navigation systems such as GPS, GLONASS, Galileo, and QZSS, as mentioned above. In this case, the position information acquired by the second acquisition unit 60 may include latitude, longitude, and altitude information. The second acquisition unit 60 can thus acquire the information about the altitude of the subject.

The second acquisition unit 60 may estimate the information about the altitude of the subject, by acquiring information of latitude and longitude. For example, with information based on a topographical map of an area including the position of the subject, the information about the altitude of the subject can be estimated from the information of the position (latitude and longitude) of the subject. To enable such estimation, position information and altitude information are associated with each other. For example, information associating position and altitude with each other may be stored in the memory 12 in the monitoring apparatus 1. In this case, the controller 10 may estimate the information about the altitude of the subject, based on the information of the position (latitude and longitude) acquired by the second acquisition unit 60. For example, the information associating position and altitude with each other may be stored in the server memory 72 (see FIG. 10). In this case, the second acquisition unit 60 may transmit the acquired information of the position (latitude and longitude) to the server 70, and the server controller 71 may estimate the information about the altitude of the subj ect.

The second acquisition unit 60 may estimate the information about the altitude of the subject, by acquiring information of the atmospheric pressure of the ambient environment like, for example, an atmospheric pressure sensor. The second acquisition unit 60 may have any structure capable of acquiring the information about at least one of the position and altitude of the subject.

Operation of the monitoring apparatus 1 illustrated in FIG. 13 will be described below.

Mountain sickness is known to be likely to occur when the subject enters a state of hypoxia, e.g. when SpO₂ decreases. In particular, the risk of developing mountain sickness is high when the altitude changes (increases or decreases) rapidly while climbing, driving an automobile on mountain roads, or the like. The degree of the risk of developing mountain sickness also depends on the path which the subject takes during climbing or the like. The degree of the risk of developing mountain sickness can also vary considerably by individual. Accordingly, the monitoring apparatus 1 according to the embodiment monitors the biological information of the subject and the information about at least one of the position and altitude of the subject, and notifies predetermined information such as urging to breath in the case where the subject is determined to have risk of developing mountain sickness.

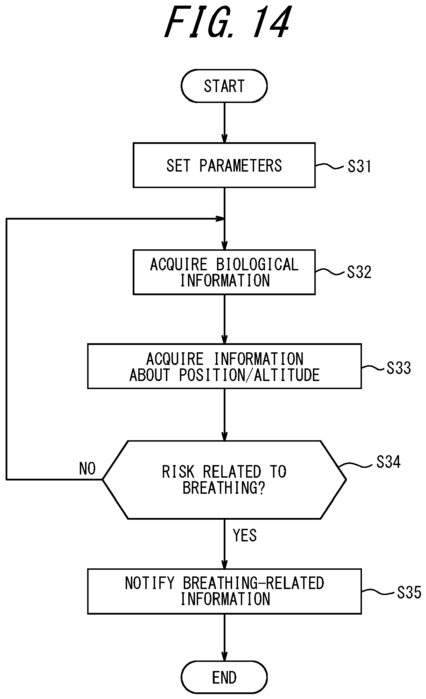

The monitoring apparatus 1 can monitor the condition of the subject according to the procedure of the flowchart illustrated in FIG. 14. As an example, suppose the monitoring apparatus 1 monitors the risk of the subject developing mountain sickness during climbing or the like.

The controller 10 sets parameters relating to control of each component in the monitoring apparatus 1 (step S31). The controller 10 may store the set parameters in the memory 12. The parameter setting in step S31 may be performed in a way same as or similar to the parameter setting in step S1 in FIG. 3.

The controller 10 acquires the biological information of the subject from the sensor 50 (step S32). In step S32, the controller 10 may control the first acquisition unit 20 to acquire the biological information of the subject from the sensor 50. That is, the first acquisition unit 20 may acquire the biological information of the subject. The type(s) of the biological information acquired may be set by the parameters. The biological information may include at least one of the breathing rate, SpO₂, oxygen saturation, body temperature, pulse rate, blood pressure, and blood flow amount of the subject.

The controller 10 acquires the information about at least one of the position and altitude of the subject from the second acquisition unit 60 (step S33). The information about the position of the subject may be, for example, information acquired by the second acquisition unit 60 from a position sensor such as a GPS module. The information about the altitude of the subject may be, for example, information of height such as elevation. The information about the altitude of the subject may be information acquired by the second acquisition unit 60 from a position sensor such as a GPS module, too. Thus, the information of the height acquired by the second acquisition unit 60 may be used as the information about the altitude of the subject. For example, in the case where the second acquisition unit 60 acquires information of an atmospheric pressure sensor, the second acquisition unit 60 may acquire information of the atmospheric pressure of the ambient environment of the monitoring apparatus 1 including the second acquisition unit 60. Thus, information of altitude (height) estimated from the information of the atmospheric pressure acquired by the second acquisition unit 60 may be used as the information about the altitude of the subject.

In FIG. 14, the process of step S32 and the process of step S33 may be performed in reverse order.

The controller 10 determines whether the subject has risk related to breathing, based on the biological information of the subject and the information about at least one of the position and altitude of the subject (step S34). Herein, the risk related to breathing (i.e. the risk related to the breathing of the subject) may be, for example, the risk of the subject developing mountain sickness. The risk related to breathing may be any of various possible problems or dangers associated with the breathing of the subject, other than mountain sickness. In step S34, the controller 10 may determine only whether the subject has risk related to breathing. In step S34, the controller 10 may determine not only whether the subject has risk related to breathing but also the degree of the risk related to the breathing of the subject (e.g. the stage of the risk or the level of the risk).

For example, the controller 10 may determine that the subject has risk related to breathing, in the case where the altitude of the subject is greater than or equal to a predetermined altitude, for example, the altitude is 2000 m or more. Moreover, for example, in the case where the altitude of the subject is 2200 m or more, the controller 10 may determine that the degree of the risk related to the breathing of the subject is higher than in the case where the altitude of the subject is 2000 m. Thus, the controller 10 may determine that the subject has risk related to breathing, based on the information about the altitude. The controller 10 may determine that the subject has risk related to breathing, in the case where the altitude of the subject changes by a predetermined amount or more, for example, the altitude increases (or decreases) by 200 m or more with respect to 1500 m. Thus, the controller 10 may determine that the subject has risk related to breathing, based on the information about the change of the altitude. The controller 10 may determine that the subject has risk related to breathing, in the case where the altitude of the subject changes by a predetermined amount or more within a predetermined time period, for example, the altitude increases (or decreases) by 300 m or more with respect to 2000 m within 30 minutes. Thus, the controller 10 may determine that the subject has risk related to breathing, based on the information about the change of the altitude within the predetermined time period. In particular, the controller 10 may determine that the subject has risk related to breathing, in the case where the change of the altitude within the predetermined time period is the predetermined amount or more.

For example, the controller 10 may estimate the moving path of the subject based on the information about the position of the subject. For example, the second acquisition unit 60 may acquire the position information of the subject from a position sensor such as a GPS module, at each predetermined timing. In this case, the controller 10 can estimate the moving path of the subject, with reference to map information around the current location of the subject. The controller 10 may then determine whether the subject has risk related to breathing, based on the estimated moving path of the subject. For example, suppose there are two paths from point A to point B, namely, a first path and a second path, when the subject moves on mountain roads on foot. In the case where the first path includes an up-slope of a steep inclination, for example, the controller 10 may determine that the risk related to the breathing of the subject is high. In the case where the second path includes only a very gentle up-slope, for example, the controller 10 may determine that the risk related to the breathing of the subject is low.

A threshold or the like used as a criterion for the determination by the controller 10 in step S34 may be stored in, for example, the memory 12 beforehand. For example, the memory 12 may store each threshold or the like for biological information as a criterion for determining whether the subject has risk related to breathing and/or the degree of the risk. For example, the memory 12 may store each threshold or the like for information about at least one of position and altitude as a criterion for determining whether the subject has risk related to breathing and/or the degree of the risk. The memory 12 may store a combination of the biological information of the subject and the information about at least one of the position and altitude of the subject, in order to determine whether the subject has risk related to breathing and/or the degree of the risk.

In step S34, the controller 10 may take, for example, the individual constitution of the subject into consideration, when determining the risk related to the breathing of the subject. Typically, the degree of the risk of developing mountain sickness can vary considerably depending on the individual constitution of the subject. Hence, for example, the controller 10 may estimate the individual constitution of the subject, based on the result of storing the biological information and/or lifelog of the subject, etc. in the memory 12. In this case, the controller 10 can determine the risk related to the breathing of the subject while taking the individual constitution of the subject into consideration.

Moreover, in step S34, the controller 10 may take, for example, the current physical condition of the subject into consideration, when determining the risk related to the breathing of the subject. Typically, the degree of the risk of developing mountain sickness can also vary considerably depending on the current physical condition of the subject. Hence, for example, the controller 10 may estimate the current physical condition of the subject, based on the result of storing the biological information and/or lifelog of the subject, etc. in the memory 12. In this case, the controller 10 can determine the risk related to the breathing of the subject while taking the current physical condition of the subject into consideration.

Thus, in the monitoring apparatus 1 according to the embodiment, the controller 10 may determine whether the subject has risk related to breathing based on the biological information of the subject and the information about at least one of the position and altitude of the subject.

In the case where the subject has no risk related to breathing (step S34: NO), the controller 10 returns to step S32. In the case where the subject has risk related to breathing (step S34: YES), the controller 10 causes the notification interface 30 to notify the subject of predetermined breathing-related information such as urging to breath (step S35). Thus, in the monitoring apparatus 1 according to the embodiment, in the case where the controller 10 determines that the subject has risk related to breathing, the controller 10 may control the notification interface 30 to notify the subject of breathing-related information.

In step S35, the controller 10 may notify, from the notification interface 30, the subject of the breathing-related information as information such as sound (speech) and/or display (warning light). In step S35, the controller 10 may notify, from the notification interface 30, tactile information such as vibration, instead of or in addition to auditory information and/or visual information. As an example, the controller 10 may notify, from the notification interface 30, the subject of a message such as "Please increase your breathing rate / Please take x deep breaths" by speech and/or display. As another example, the controller 10 may notify, from the notification interface 30, the subject of a message such as "Please increase your breathing rate and rest awhile" by speech and/or display. As yet another example, the controller 10 may notify, from the notification interface 30, the subject of a message such as "You have risk of mountain sickness" by speech and/or display.

In step S35, for example, the controller 10 may notify the server 70 of information that the subject has risk related to breathing. In step S35, for example, the controller 10 may notify an external medical institution or clinic of information that the subject has risk related to breathing.

Hence, the monitoring apparatus 1 according to the embodiment can reduce the risk of the subject developing mountain sickness during climbing or the like. The monitoring apparatus 1 according to the embodiment can therefore reduce the risk related to the physical condition of the user.

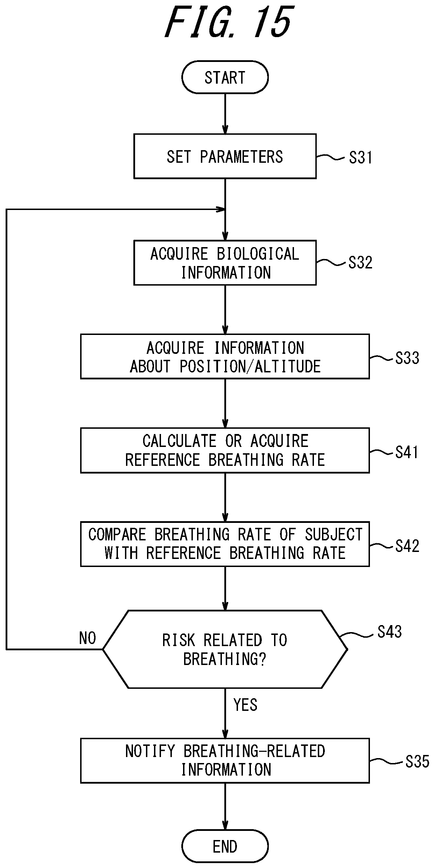

The monitoring apparatus 1 may monitor the condition of the subject according to the procedure of the flowchart illustrated in FIG. 15. As an example, suppose the monitoring apparatus 1 monitors the risk of the subject developing mountain sickness during climbing or the like in FIG. 15, as in FIG. 14.

The procedure of the flowchart in FIG. 15 differs from the procedure of the flowchart in FIG. 14 in that the processes of steps S41 and S42 are added and the process of step S34 is changed to the process of step S42.

The controller 10 sets parameters relating to control of each component in the monitoring apparatus 1 (step S31). The controller 10 may store the set parameters in the memory 12. The parameter setting in step S31 may be performed in a way same as or similar to the parameter setting in step S1 in FIG. 3.

The controller 10 acquires the biological information of the subject from the sensor 50 (step S32). The controller 10 acquires the information about the position and/or altitude of the subject from the second acquisition unit 60 (step S33). The processes of steps S32 and S33 may be the same as or similar to the processes of steps S32 and S33 illustrated in FIG. 14. In FIG. 15, the process of step S32 and the process of step S33 may be performed in reverse order, as in FIG. 14.

The controller 10 then calculates or acquires a reference breathing rate (step S41). The reference breathing rate calculated or acquired in step S41 is a breathing rate to be compared with the breathing rate of the subject in the following step S42. For example, the breathing rate and the reference breathing rate may each be the number of breaths per minute [breaths/min] or the average number of breaths per minute [breaths/min]. The reference breathing rate calculated or acquired in step S41 will be described in detail below.

As mentioned above, the controller 10 may estimate the moving path of the subject based on the information about the position of the subject. The controller 10 may estimate the moving path of the subject based on the information about the altitude of the subject instead of or in addition to the information about the position of the subject. That is, the controller 10 may estimate the moving path of the subject based on the information about at least one of the position and altitude of the subject. In the case where the moving path of the subject can be estimated, the controller 10 may calculate the breathing rate corresponding to the moving path as the reference breathing rate. In this case, the controller 10 may calculate, as the reference breathing rate, such a breathing rate that does not increase the risk related to the breathing of the subject, based on the distance of movement of the subject in the estimated moving path, the time for moving the distance, etc. The controller 10 may calculate, as the reference breathing rate, such a breathing rate that does not increase the risk related to the breathing of the subject, based on various environmental conditions such as gradients, topography, obstacles, and oxygen concentration in the atmosphere included in the estimated moving path.

For example, algorithms for calculating such reference breathing rates may be stored in the memory 12 beforehand, or acquired from an apparatus such as the server 70 via the network 80 illustrated in FIG. 9.

The reference breathing rate may be, for example, a breathing rate unique (typical) to the moving path as the breathing rate corresponding to the moving path, without depending on the subject. The controller 10 may calculate the reference breathing rate based on, for example, information input by the subject through the input interface 40.

Alternatively, for example, the reference breathing rate may be a breathing rate that varies depending on the subject. For example, the reference breathing rate may be a breathing rate obtained by correcting the breathing rate stored in association with the corresponding moving path based on at least one of the subject's age, sex, physical fitness, exercise history, climbing history, current physical condition, and the like. In such a case, for example, the controller 10 may calculate the reference breathing rate based on the biological information of the subject acquired in step S32. To do so, the controller 10 may refer to the biological information of the subject stored in the memory 12. The controller 10 may refer to the past biological information of the subject stored in the memory 12. The controller 10 may calculate the reference breathing rate based on, for example, information input by the subject through the input interface 40.

The reference breathing rate may be, for example, a breathing rate calculated based on the past information of the subject. For example, suppose no risk related to the breathing of the subject was detected when the subject was breathing at a predetermined breathing rate while moving on mountain roads of a predetermined altitude in the past. In this case, the predetermined breathing rate at which the subject was breathing may be assumed to be suitable as the reference breathing rate. Suppose risk related to the breathing of the subject was detected when the subject was breathing at a predetermined breathing rate while moving on mountain roads of a predetermined altitude in the past. In this case, the predetermined breathing rate at which the subject was breathing may be assumed to be not suitable as the reference breathing rate. Here, whether the subject has risk related to breathing and/or the degree of the risk may be determined from the biological information of the subject (or biological information history). Whether the subject has risk related to breathing and/or the degree of the risk may be determined based on information (e.g. subjective symptoms) input by the subject through the input interface 40.

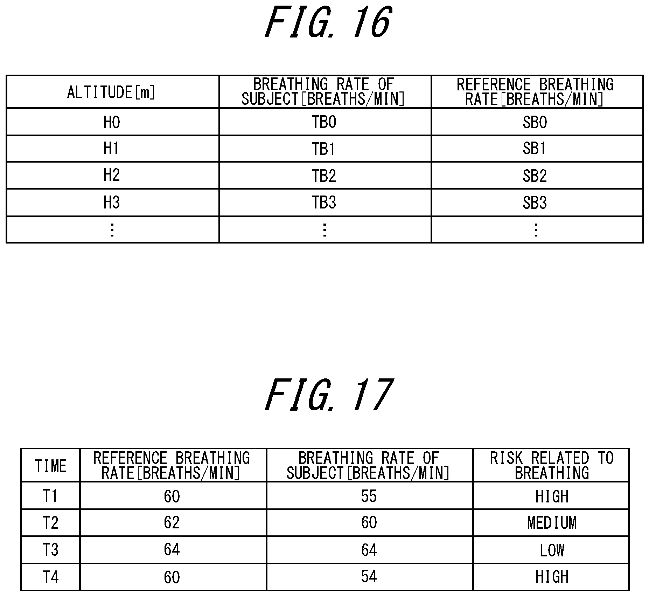

The memory 12 may store the biological information of the subject in association with the information about at least one of the position and altitude of the subject, in order to calculate the reference breathing rate based on the past information of the subject. For example, the memory 12 may store the breathing rate of the subject in association with each altitude, as illustrated in FIG. 16. FIG. 16 is a diagram illustrating an example in which the information about the altitude of the subject and the breathing rate of the subject are stored in association with each other.

For example, in the case where the breathing rate of the subject is TBO [breaths/min] when the altitude of the subject is H0 [m], the controller 10 may store these information in the memory 12 in association with each other. In this case, the controller 10 may calculate a reference breathing rate SB0 when the altitude of the subject is H0 [m], based on the breathing rate TBO [breaths/min] of the subject. In the case where no risk related to the breathing of the subject is detected when the breathing rate of the subject is TB0, the controller 10 may set the breathing rate TBO of the subject as the reference breathing rate SB0. In the case where risk related to the breathing of the subject is detected when the breathing rate of the subject is TB0, the controller 10 may correct the breathing rate TBO of the subject based on a predetermined algorithm and set the corrected breathing rate as the reference breathing rate SB0. The controller 10 may then store the calculated reference breathing rate SB0 and the altitude H0 of the subject in the memory 12 in association with each other.

Subsequently, when the altitude of the subject is H0 [m], the controller 10 may calculate the reference breathing rate as SB0 by referring to the memory 12. Thus, the controller 10 may calculate the reference breathing rate based on the biological information of the subject stored in association with the information about at least one of the position and altitude of the subj ect.

For example, in the case where the breathing rate of the subject is TB1, TB2, and TB3 [breaths/min] when the altitude of the subject is HI, H2, and H3 [m] respectively, the controller 10 may store these information in the memory 12 in association with each other. Here, the altitudes HI, H2, and H3 [m] of the subject may include different altitudes. Thus, the memory 12 may store the biological information of the subject in association with the information about a corresponding one of a plurality of different altitudes of the subject.

In this case, as mentioned above, the controller 10 may calculate the reference breathing rates SB1, SB2, and SB3 when the altitude of the subject is HI, H2, and H3 [m] respectively, based on the breathing rates TB1, TB2, and TB3 [breaths/min] of the subject. Subsequently, when the altitude of the subject is HI, H2, and H3 [m], the controller 10 may calculate the reference breathing rate as SB1, SB2, and SB3 respectively, by referring to the memory 12.

The controller 10 may, for example based on the breathing rates TB1, TB2, and TB3 of the subject respectively corresponding to the altitudes HI, H2, and H3 of the subject, calculate (estimate) a reference breathing rate corresponding to an altitude of the subject other than the altitudes HI, H2, and H3. For example, assuming that the reference breathing rate corresponding to the altitude of the subject changes linearly, the controller 10 may calculate the reference breathing rate by the least-squares method. Subsequently, when the altitude of the subject is other than HI, H2, and H3 [m], for example, the controller 10 can calculate a relatively appropriate reference breathing rate. Thus, the controller 10 may calculate the reference breathing rate based on the biological information of the subject stored in association with the information about a corresponding one of a plurality of different altitudes of the subject.

While some reference breathing rate calculation (estimation) methods have been described above, the reference breathing rate may be calculated (estimated) based on any of other various algorithms and the like.

The controller 10 may acquire the reference breathing rate from an apparatus such as the server 70 located outside the monitoring apparatus 1 via the network 80 illustrated in FIG. 9 as an example, without calculating the reference breathing rate. In such a case, the server 70 may store information of each reference breathing rate so as to respond to a request from the monitoring apparatus 1. The server 70 may, in response to a request from the monitoring apparatus 1, acquire information of a desired reference breathing rate from another server or the like.

After calculating or acquiring the reference breathing rate in step S41, the controller 10 compares the calculated or acquired reference breathing rate with the breathing rate of the subject (step S42). The breathing rate of the subject compared with the reference breathing rate in step S42 may be information obtained based on the biological information of the subject acquired in step S32. For example, in the case where the biological information of the subject acquired in step S32 includes the breathing rate of the subject (or information corresponding to the breathing rate), the controller 10 may use the information as the breathing rate of the subject. In the case where the biological information of the subject acquired in step S32 does not include the breathing rate of the subject (or information corresponding to the breathing rate), the controller 10 may estimate the breathing rate of the subject based on at least one of other types of biological information of the subject acquired in step S32.

The controller 10 determines whether the subject has risk related to breathing, based on the result of the comparison in step S42 (step S43). For example, in the case where the breathing rate of the subject is greater than or equal to the reference breathing rate in step S43, the controller 10 may determine that the subject has no risk related to breathing or the risk is low. In the case where the breathing rate of the subject is less than (i.e. below) the reference breathing rate in step S43, the controller 10 may determine that the subject has risk related to breathing.

In the case where the subject has no risk related to breathing or the risk is low (step S43: NO), the controller 10 returns to step S32. In the case where the subject has risk related to breathing (step S43: YES), the controller 10 causes the notification interface 30 to notify the subject of predetermined breathing-related information such as urging to breath (step S35). Thus, the monitoring apparatus 1 according to the embodiment can reduce the risk of the subject developing mountain sickness during climbing or the like. The monitoring apparatus 1 according to the embodiment can therefore reduce risk related to the physical condition of the user.

In the case where the controller 10 determines that the subject has risk related to breathing in step S43, the controller 10 may also determine the degree of the risk. In this case, for example, the controller 10 may determine that the risk related to the breathing of the subject is high, depending on the extent to which the breathing rate of the subject is less than (i.e. below) the reference breathing rate. For example, in the case where the breathing rate of the subject is less than the reference breathing rate but the difference calculated by subtracting the breathing rate of the subject from the reference breathing rate is not greater than a first threshold, the controller 10 may determine that the risk related to the breathing of the subject is low. For example, in the case where the difference calculated by subtracting the breathing rate of the subject from the reference breathing rate is greater than the first threshold but not greater than a second threshold, the controller 10 may determine that the risk related to the breathing of the subject is medium. For example, in the case where the difference calculated by subtracting the breathing rate of the subject from the reference breathing rate is greater than the second threshold, the controller 10 may determine that the risk related to the breathing of the subject is high.

FIG. 17 is a diagram illustrating an example in which the first threshold is 1 and the second threshold is 4. At time T1, the calculated reference breathing rate is 60 [breaths/min], and the acquired breathing rate of the subject is 55 [breaths/min], as illustrated in FIG. 17. In this case, the difference calculated by subtracting the breathing rate of the subject from the reference breathing rate is 5, which is greater than the second threshold. The controller 10 accordingly determines the risk related to the breathing of the subject to be "high".

At time T2, the calculated reference breathing rate is 62 [breaths/min], and the acquired breathing rate of the subject is 60 [breaths/min]. In this case, the difference calculated by subtracting the breathing rate of the subject from the reference breathing rate is 2, which is greater than the first threshold but is not greater than the second threshold. The controller 10 accordingly determines the risk related to the breathing of the subject to be "medium". At time T3, the difference calculated by subtracting the breathing rate of the subject from the reference breathing rate is 0, which is not greater than the first threshold. The controller 10 accordingly determines the risk related to the breathing of the subject to be "low". At time T4, the difference calculated by subtracting the breathing rate of the subject from the reference breathing rate is 6, which is greater than the second threshold. The controller 10 accordingly determines the risk related to the breathing of the subject to be "high". When determining the risk related to the breathing of the subject, the controller 10 may use a number calculated by dividing the breathing rate of the subject by the reference breathing rate, or a number calculated by dividing the reference breathing rate by the breathing rate of the subject. For example, when determining the risk related to the breathing of the subject, the controller 10 may determine that the risk related to the breathing is high in the case where the number calculated by dividing the breathing rate of the subject by the reference breathing rate is not greater than a predetermined value.

In the case where the subject has no risk related to breathing as a result of such determination (step S43: NO), the controller 10 may return to step S32. In the case where the subject has risk related to breathing (step S43: YES), the controller 10 may cause the notification interface 30 to notify, for example, the subject of predetermined breathing-related information such as urging to breath, depending on the determined degree of the risk (step S35).

In such a case, in step S35, the controller 10 may notify the subject of the breathing-related information from the notification interface 30, by changing sound (speech) and/or display (warning light) depending on the determined degree of the risk. For example, the controller 10 may increase the volume of sound (speech) and/or the intensity of display (warning light) notified from the notification interface 30, as the determined degree of the risk increases. For example, the controller 10 may increase the level of seriousness of the message represented by the speech and/or display notified to the subject from the notification interface 30, as the determined degree of the risk increases.

Thus, in the monitoring apparatus 1 according to the embodiment, the controller 10 may determine whether the subject has risk related to breathing, by comparing the breathing rate of the subject obtained based on the biological information of the subject with the predetermined reference breathing rate. For example, in the case where the breathing rate of the subject is less than the reference breathing rate by at least a predetermined value, the controller 10 may determine that the subject has risk related to breathing. Hence, the monitoring apparatus 1 according to the embodiment can reduce the risk of the subject developing mountain sickness during climbing or the like. The monitoring apparatus 1 according to the embodiment can therefore reduce the risk related to the physical condition of the user.

When determining whether the subject has risk related to breathing in step S43, the controller 10 may take the exercise state of the subject into consideration. For example, the risk related to the breathing of the subject is likely to be different between in a state in which the subject is doing hard exercise and in a state in which the subject is doing light exercise, even though the environment is the same. Accordingly, for example, even when the controller 10 determines that the subject has no risk related to breathing or the risk is low in step S43, if the subject is doing hard exercise, the controller 10 may determine that the subject has risk related to breathing. Here, the exercise state of the subject may be estimated based on various information in the monitoring apparatus 1. For example, the controller 10 may estimate the exercise state of the subject, based on the biological information of the subject acquired by the first acquisition unit 20. The controller 10 may estimate the exercise state of the subject, based on the information about the position and/or altitude of the subject acquired by the second acquisition unit 20. The controller 10 may estimate the exercise state of the subject, based on at least one of the foregoing various types of information.

Thus, in the monitoring apparatus 1 according to an embodiment, the controller 10 may determine whether the subject has risk related to breathing based on the exercise state of the subject. In this case, the controller 10 may estimate the exercise state of the subject, based on at least one of the biological information of the subject, the information about the position of the subject, and the information about the altitude of the subject.

Although the embodiments according to the present disclosure have been described by way of the drawings and examples, various changes or modifications may be easily made by those of ordinary skill in the art based on the present disclosure. Such various changes or modifications are therefore included in the scope of the present disclosure. For example, the functions included in the components, steps, etc. may be rearranged without logical inconsistency, and a plurality of components, steps, etc. may be combined into one component, step, etc. and a component, step, etc. may be divided into a plurality of components, steps, etc. Although apparatuses have been mainly described in the embodiments according to the present disclosure, the embodiments according to the present disclosure can also be implemented as methods including the steps executed by the components included in the apparatuses. The embodiments according to the present disclosure can also be implemented as methods or programs executed by processors included in the apparatuses or storage media storing such programs, which are also included in the scope of the present disclosure. While some embodiments and examples of the present disclosure have been described above by way of drawings, various changes and modifications may be easily made by those of ordinary skill in the art based on the present disclosure. Such changes and modifications are therefore included in the scope of the present disclosure. For example, the functions included in the functional parts, etc. may be rearranged without logical inconsistency, and a plurality of functional parts, etc. may be combined into one functional part, etc. and a functional part, etc. may be divided into a plurality of functional parts, etc. Moreover, each of the disclosed embodiments is not limited to the strict implementation of the embodiment, and features may be combined or partially omitted as appropriate. That is, various changes and modifications may be made to the presently disclosed techniques by those of ordinary skill in the art based on the present disclosure. Such changes and modifications are therefore included in the scope of the present disclosure. For example, functional parts, means, steps, etc. in each embodiment may be added to another embodiment without logical inconsistency, or replace functional parts, means, steps, etc. in another embodiment. In each embodiment, a plurality of functional parts, means, steps, etc. may be combined into one functional part, means, step, etc., and a functional part, means, step, etc. may be divided into a plurality of each functional parts, means, steps, etc. Moreover, each of the disclosed embodiments is not limited to the strict implementation of the embodiment, and features may be combined or partially omitted as appropriate.

Terms such as "first" and "second" in the present disclosure are identifiers for distinguishing components. Components distinguished by terms such as "first" and "second" in the present disclosure may have their numbers interchanged with each other. For example, the identifier "first" of the "first information" and the identifier "second" of the "second information" may be interchanged with each other. The identifiers are replaced with each other simultaneously. The components are distinguishable even after their identifiers are interchanged. The identifiers may be omitted. Components from which identifiers are omitted are distinguished by reference signs. Description of identifiers such as "first" and "second" in the present disclosure alone should not be used for interpretation of order of components or reasoning based on one identifier being smaller than another identifier.

The foregoing embodiments are not limited to implementation as the monitoring apparatus 1. For example, the foregoing embodiments may be implemented as a monitoring method of an apparatus such as the monitoring apparatus 1. For example, the foregoing embodiments may be implemented as a monitoring program to be executed by an apparatus such as the monitoring apparatus 1.

### REFERENCE SIGNS LIST

- 1: monitoring apparatus
- 10: controller
- 12: memory
- 20: first acquisition unit
- 30: notification interface
- 40: input interface
- 50: sensor
- 60: second acquisition unit
- 70: server
- 71: server controller
- 711: server processor
- 72: server memory
- 73: server communication interface
- 80: network
- 201, 201L, 201R: measurement unit
- 203, 203L, 203R: holder
- 205, 205L, 205R: housing
- 207: connector
- 209: sensor communication interface
- 400, 500: data structure

## Claims

1. A monitoring apparatus comprising:
a first acquisition unit configured to acquire biological information of a subject;
a second acquisition unit configured to acquire information about at least one of a position and an altitude of the subject;
a notification interface configured to notify the subject of predetermined information; and
a controller configured to control the notification interface to notify the subject of breathing-related information, in the case of determining that the subject has risk related to breathing based on the biological information of the subject and the information about at least one of the position and the altitude of the subject.

2. The monitoring apparatus according to claim 1, wherein the controller is configured to determine whether the subject has the risk related to the breathing, by comparing a breathing rate of the subject obtained based on the biological information of the subject with a predetermined reference breathing rate.

3. The monitoring apparatus according to claim 2, wherein the controller is configured to determine that the subject has the risk related to the breathing, in the case where the breathing rate of the subject is less than the reference breathing rate by at least a predetermined value.

4. The monitoring apparatus according to claim 2 or 3, wherein the controller is configured to estimate a moving path of the subject based on the information about at least one of the position and the altitude of the subject, and calculate a breathing rate corresponding to the moving path as the reference breathing rate.

5. The monitoring apparatus according to claim 2 or 3, comprising
a memory configured to store the biological information of the subj ect,
wherein the controller is configured to calculate the reference breathing rate, based on the biological information of the subject stored in the memory.

6. The monitoring apparatus according to claim 5, wherein the memory is configured to store the biological information of the subject in association with the information about at least one of the position and the altitude of the subject, and
the controller is configured to calculate the reference breathing rate, based on the biological information of the subject stored in association with the information about at least one of the position and the altitude of the subj ect.

7. The monitoring apparatus according to claim 5, wherein the memory is configured to store the biological information of the subject in association with information about each of a plurality of different altitudes of the subject, and
the controller is configured to calculate the reference breathing rate, based on the biological information of the subject stored in association with the information about each of the plurality of different altitudes of the subject.

8. The monitoring apparatus according to claim 2 or 3, wherein the controller is configured to acquire the reference breathing rate from outside the monitoring apparatus.

9. The monitoring apparatus according to any of claims 1 to 8, wherein the controller is configured to estimate an exercise state of the subject based on at least one of the biological information of the subject, information about the position of the subject, and information about the altitude of the subject, and determine whether the subject has the risk related to the breathing based on the exercise state of the subject.

10. The monitoring apparatus according to any of claims 1 to 8, wherein the biological information includes at least one of a breathing rate, an oxygen saturation, a pulse rate, and a body temperature of the subject.

11. A monitoring method comprising:
acquiring biological information of a subject;
acquiring information about at least one of a position and an altitude of the subject;
notifying the subject of predetermined information; and
controlling to notify the subject of breathing-related information, in the case of determining that the subject has risk related to breathing based on the biological information of the subject and the information about at least one of the position and the altitude of the subject.

12. A monitoring program for causing a computer to:
acquire biological information of a subject;
acquire information about at least one of a position and an altitude of the subject;
notify the subject of predetermined information; and
control to notify the subject of breathing-related information, in the case of determining that the subject has risk related to breathing based on the biological information of the subject and the information about at least one of the position and the altitude of the subject.
